# EUROPEAN PATENT APPLICATION

(11) **EP 3 943 136 A1**
(43) Date of publication of application: **26.01.2022**
(21) Application number: 21187542.2
(22) Date of filing: 23.07.2021
(51) Int. Cl.: A61M 16/00, A61M 16/20

(54) **PATIENT VENTILATOR AND CONTROLLER THEREFOR**

(30) Priority: 24.07.2020 US 202063055940 P
(71) Applicant: Pratt & Whitney Canada Corp., Longueuil, Québec J4G 1A1 (CA); Belleville, Francois, (01BE5) Longueuil, Québec J4G 1A1 (CA)
(72) Inventor: BELLEVILLE, Francois, (01BE5) Longueuil, Québec J4G 1A1 (CA); THOMASSIN, Jean, (01BE5) Longueuil, J4G 1A1 (CA)
(74) Representative: Dehns

(57) **Abstract**

There is provided a ventilator (100) to ventilate an airway (112) of a patient. The ventilator (100) has a conduit (114) having an inlet (114a) and an outlet (114b), said outlet (114b) configured to be connected to said airway (112); a gas delivery element (116) in fluid communication with said inlet (114a), said gas delivery element (116) configured to deliver air in a sequence of ventilation cycles, each ventilation cycle defined by a corresponding tidal volume to be delivered to said airway (112) via said conduit (114), each tidal volume corresponding to a difference between a start volume and an end volume of said gas delivery element (116) for that ventilation cycle; a pressure sensor (120) monitoring pressure within said conduit (114); a controller (122) communicatively coupled to said gas delivery element (116) and said pressure sensor (120), said controller (122) performing reducing said tidal volume between a first ventilation cycle and a successive second ventilation cycle contingent upon said pressure exceeding a pressure threshold in said first ventilation cycle.

## Description

### TECHNICAL FIELD

The application relates generally to ventilators, more particularly, to patient ventilators configured to ventilate patient airways.

### BACKGROUND OF THE ART

Ventilators are machines that provide mechanical ventilation by delivering breathable air into and evacuating it out from patients' lungs. Such ventilators are typically used to deliver breaths to a patient who is physically unable to breathe, or who breathes insufficiently. Ventilators can be computerized microprocessor-controlled machines configured to deliver, in a sequence of ventilation cycles, the necessary volume of breathable air to a patient, a quantity generally referred to as a tidal volume. Although existing ventilators are satisfactory to a certain degree, there always remains room for improvement.

### SUMMARY

In accordance with a first aspect of the present disclosure, there is provided a ventilator to ventilate an airway of a patient, the ventilator comprising: a conduit having an inlet and an outlet, said outlet being configured to be connected to said airway; a gas delivery element in fluid communication with said inlet of said conduit, said gas delivery element being configured to deliver air in a sequence of ventilation cycles, each ventilation cycle being defined by a corresponding tidal volume to be delivered to said airway via said conduit, each tidal volume corresponding to a difference between a start volume and an end volume of said gas delivery element for that ventilation cycle; a pressure sensor configured to monitor pressure within said conduit; a controller communicatively coupled to said gas delivery element and said pressure sensor, said controller having a processor and a non-transitory memory having stored thereon instructions that when executed by said processor perform the step of reducing said tidal volume between a first ventilation cycle and a successive second ventilation cycle contingent upon said pressure exceeding a pressure threshold in said first ventilation cycle.

In accordance with a second aspect of the present disclosure, there is provided a method of ventilating an airway of a patient, said method comprising: monitoring an airway pressure of said patient; delivering a first ventilation cycle of air to said airway, said first ventilation cycle defined by a first tidal volume and generating pressure variations in said airway; and delivering a second ventilation cycle of air to said airway, said second ventilation cycle subsequent to said first ventilation cycle and defined by a second tidal volume, said second tidal volume being lesser than said first tidal volume contingent upon said airway pressure exceeding an airway pressure threshold during said first ventilation cycle.

In accordance with a third aspect of the present disclosure, there is provided a controller configured for controlling a patient ventilator based on airway pressure monitoring, said controller having a processor and a non-transitory memory having stored thereon instructions that when executed by said processor perform the step of: reducing a tidal volume between a first ventilation cycle and a successive second ventilation cycle contingent upon an airway pressure measurement exceeding a pressure threshold during said first ventilation cycle.

### DESCRIPTION OF THE DRAWINGS

Reference is now made to the accompanying figures in which:
Fig. 1 is a schematic view of an example of a ventilator, shown with a conduit, a gas delivery element, a pressure sensor and a controller, in accordance with one or more embodiments;
Fig. 2A is a schematic sectional view of the gas delivery element of Fig. 1, shown with a moving element at a start position, in accordance with one or more embodiments;
Fig. 2B is a schematic sectional view of the gas delivery element of Fig. 1, shown with the moving element at an end position, in accordance with one or more embodiments;
Fig. 3 is a schematic view of an example of a computing device of the controller of Fig. 1, in accordance with one or more embodiments; and
Fig. 4 is a flow chart of a method of ventilating an airway of a patient using the ventilator of Fig. 1, in accordance with one or more embodiments;
Fig. 5A is a graph showing tidal volume being delivered as a function of a sequence of ventilation cycles, showing the delivered tidal volume being reduced from a first tidal volume to a second tidal volume, and then being increased back to the first tidal volume, in accordance with one or more embodiments;
Fig. 5B is a graph showing airway pressure during the sequence of ventilation cycles of Fig. 5A, showing a ventilation cycle in which airway pressure exceeds a given airway pressure threshold during a given ventilation cycle;
Fig. 6A is a graph showing tidal volume being delivered as a function of a sequence of ventilation cycles, showing the delivered tidal volume being reduced from a first tidal volume to a second tidal volume, and then maintained for a number of ventilation cycles prior to being increased back to the first tidal volume, in accordance with one or more embodiments;
Fig. 6B is a graph showing airway pressure during the sequence of ventilation cycles of Fig. 6A, showing a ventilation cycle in which airway pressure exceeds a given airway pressure threshold during more than one ventilation cycle;
Fig. 7A is a graph showing tidal volume being delivered as a function of a sequence of ventilation cycles, showing the delivered tidal volume being reduced incrementally until a minimal tidal volume is reached, in accordance with one or more embodiments;
Fig. 7B is a graph showing airway pressure during the sequence of ventilation cycles of Fig. 7A, showing a ventilation cycle in which airway pressure exceeds a given airway pressure threshold during more than one ventilation cycle;
Fig. 8 is a schematic view of another example of a ventilator, with a gas delivery unit and a solenoid valve, shown with an example gas delivery element and an example solenoid valve, in accordance with one or more embodiments;
Fig. 8A is an enlarged view of the gas delivery element of Fig. 8, in accordance with one or more embodiments; and
Fig. 8B is an enlarged view of the solenoid valve of Fig. 8, in accordance with one or more embodiments.

### DETAILED DESCRIPTION

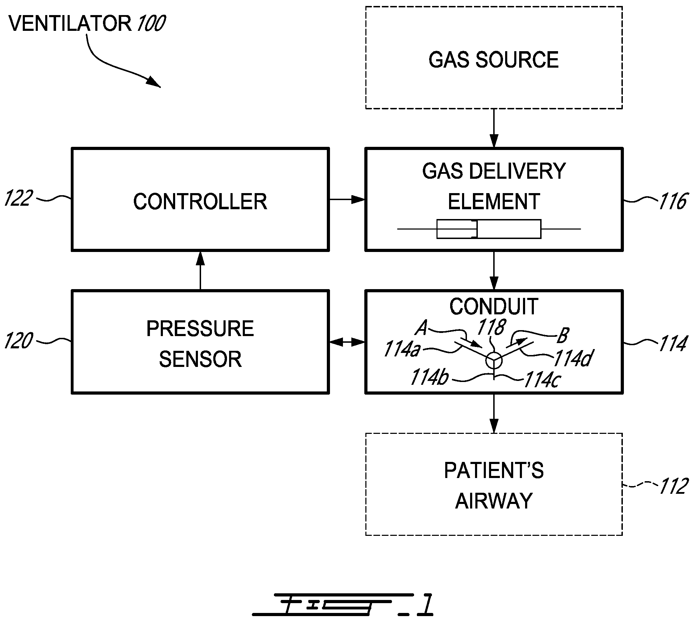

Fig. 1 shows an example of a ventilator 100 to ventilate an airway 112 of a patient, in accordance with an embodiment. As depicted, the ventilator 100 has a conduit 114 with a fresh air inlet 114a and a fresh air outlet 114b. During use of the ventilator 100, the fresh air outlet 114b of the conduit 114 is configured to be connected to the airway 112 of the patient.

The ventilator 100 has a gas delivery element 116 which is in fluid communication with the fresh air inlet 114a of the conduit 114. It is intended that the gas delivery element 116 is configured to deliver air to the conduit 114, and therefore to the patient's airway 112, in a sequence of ventilation cycles of air. Each ventilation cycle of air is defined by a corresponding tidal volume Vt to be delivered to the airway 112 of the patient via the conduit 114. Each tidal volume Vt corresponds to a difference between a start volume Vs and an end volume Ve of the gas delivery element 114 for the corresponding ventilation cycle.

It is expected that once a given tidal volume Vt of fresh air is delivered to the patient's airway 112, a corresponding volume of used air may be evacuated from the patient's airway 112 via the conduit 114. More specifically, in this embodiment, used air may be channeled from a used air inlet 114c of the conduit 114 towards a used air outlet 114d. As shown in this specific example, the fresh air outlet 114b may coincide with the used air inlet 114c when a valve 118 (e.g., a solenoid valve) is used to determine whether fresh air is to be delivered from the ventilator 120 to the patient's airway 112 in a first air flow direction A or used air is to be evacuated from the patient's airway 112 towards the ventilator 100 in a second air flow B direction opposite to the first air flow direction A.

It is noted that each ventilation cycle generates corresponding pressure variations in the airway. As such, the ventilator 100 is provided with a pressure sensor 120 configured to monitor an airway pressure Paw within the conduit 114, the monitored airway pressure Paw being indicative of a pressure within the patient's airway 112. The pressure sensor 120 is generally provided anywhere within the ventilator 100, provided that is measures the airway pressure Paw as fresh air is delivered to the patient's airway 112. For instance, the pressure sensor 120 may be positioned within the fresh air inlet 114a, within the fresh air outlet 114b, downstream from the conduit 114, or upstream from the conduit 114. In some embodiments, a single pressure sensor may be positioned at the fresh air outlet or at the used air inlet, downstream from the valve thereby monitoring the airway pressure regardless of the position of the valve 118. In some other embodiments, more than one pressure sensor can be provided. For instance, a first pressure sensor may be positioned within the fresh air inlet 114a to monitor the airway pressure Paw as fresh air is delivered to the patient's airway 112 when the valve position is open, and a second pressure sensor may be positioned within the used air outlet 114d to monitor the airway pressure Paw as used air is evacuated from the patient's airway 112 when the valve position is closed. As such, airway pressure Paw may be monitored throughout entire ventilation cycles, during both fresh air delivery and used air evacuation.

The ventilator 100 has a controller 122 which is communicatively coupled to the gas delivery element 116 and to the pressure sensor 120. Such a communicative coupling may be wired, wireless, or a combination of both depending on the embodiment. As described below, the controller 122 has a processor and a non-transitory memory which has stored thereon instructions that when executed by the processor perform some predetermined steps. More specifically, the controller 122 is configured to reduce the tidal volume Vt1 between a first ventilation cycle and a successive second ventilation cycle contingent upon the monitored airway pressure Paw exceeding a pressure threshold Pthres in the first ventilation cycle (Paw >Pthres) .

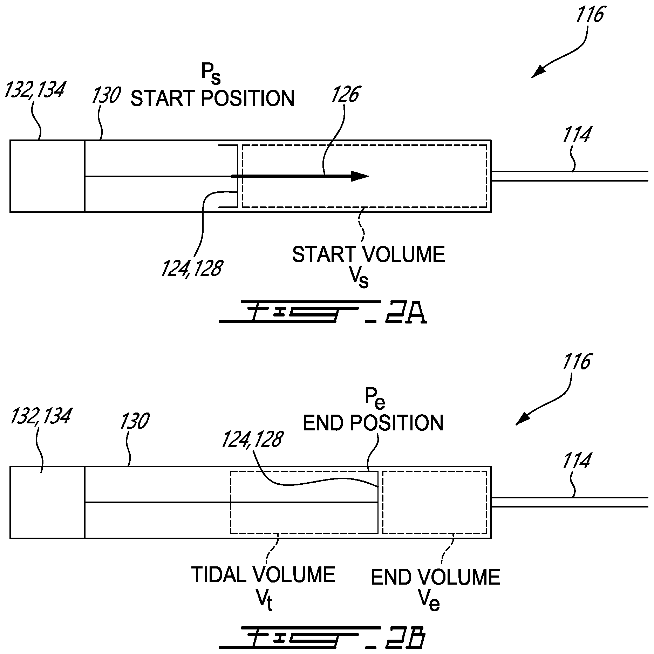

Figs. 2A and 2B show an example of a gas delivery element 116. As depicted, the gas delivery element 116 has a moving element 124 displaceable between a start position Ps corresponding to the start volume Vs and an end position Pe corresponding to the end volume Ve. More specifically, the moving element 124 is displaced at the start position Ps in Fig. 2A, which thereby defines the start volume Vs. In Fig. 2B, the moving element 124 has been moved along an axial direction 126 to the end position Pe which defines the end volume Ve. As shown, the difference between the start and end volumes corresponds to the tidal volume Vt that is to be delivered to the patient's airway via the conduit 114. In some embodiments, the moving element 124 is provided in the form of a piston 128 which is movable within a cylinder 130. In these embodiments, the start and end positions Ps and Pe correspond to axial positions of the cylinder 130. As shown, the moving element 124 may be moved between these axial position using a an actuator 132. In some embodiments, the actuator 132 is provided in the form of an electric linear actuator 134.

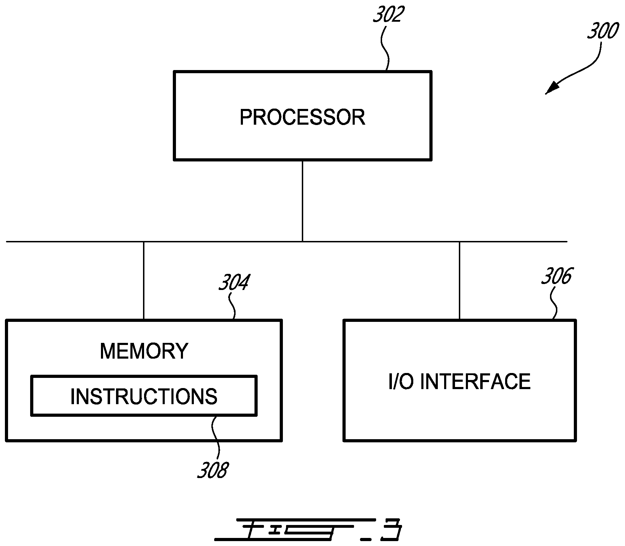

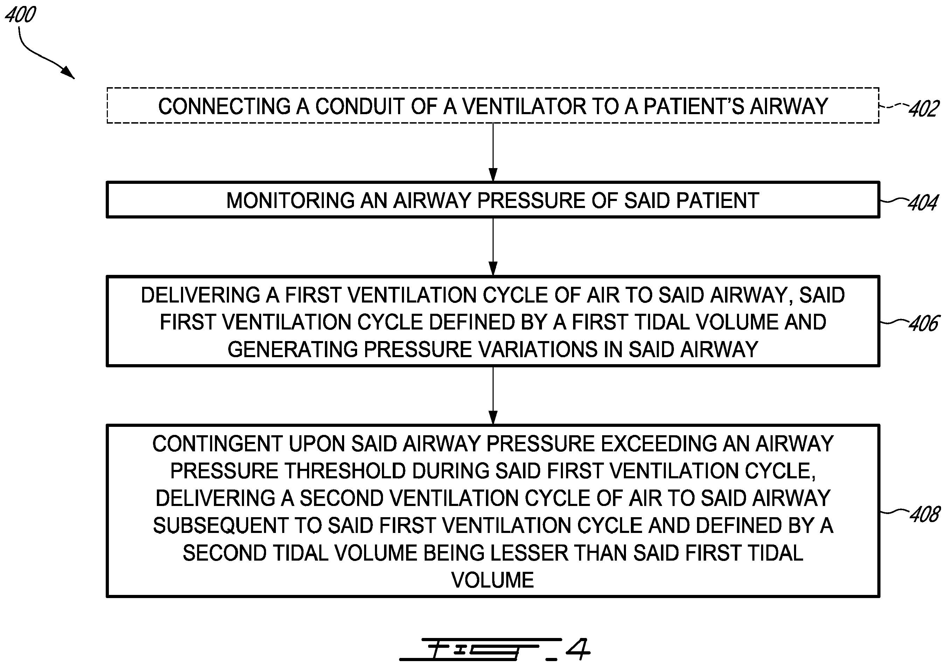

The controller 122 can be provided as a combination of hardware and software components. The hardware components can be implemented in the form of a computing device 300, an example of which is described with reference to Fig. 3. Moreover, the software components of the controller 122 can be implemented in the form of a software application adapted to perform steps of a method of ventilating a patient's airway, an example of which is described with reference to the flow chart of Fig. 4.

Referring to Fig. 3, the computing device 300 can have a processor 302, a non-transitory memory 304, and I/O interface 306. Instructions 308 for performing method steps (described above and below) can be stored on the memory 304 and accessible by the processor 302.

The processor 302 can be, for example, a general-purpose microprocessor or microcontroller, a digital signal processing (DSP) processor, an integrated circuit, a field programmable gate array (FPGA), a reconfigurable processor, a programmable read-only memory (PROM), or any combination thereof.

The memory 304 can include a suitable combination of any type of computer-readable memory that is located either internally or externally such as, for example, random-access memory (RAM), read-only memory (ROM), compact disc read-only memory (CDROM), electro-optical memory, magneto-optical memory, erasable programmable read-only memory (EPROM), and electrically-erasable programmable read-only memory (EEPROM), Ferroelectric RAM (FRAM) or the like. The memory 304 may have stored thereon values such as one or more airway pressure threshold, some tidal volumes, volume increase or decrease increments, a minimal tidal volume, and the like.

Each I/O interface 306 enables the computing device 300 to interconnect with one or more input devices, such as keyboard(s) and mouse(s), or with one or more output devices such as monitor(s), remote network(s). For instance, such input devices can be used as a user interface which can be used to input an initial tidal volume which is to be delivered to the patient's airway. The initial tidal volume, which is often referred to as the first tidal volume herein, can be set by a health professional via the user interface. The initial tidal volume associated with the patient may depend on the patient's characteristics (e.g., height, weight, age, gender), the condition of his/her airway and the like.

Each I/O interface 306 enables the controller 122 to communicate with other components, to exchange data with other components, to access and connect to network resources, to serve applications, and perform other computing applications by connecting to a network (or multiple networks) capable of carrying data including the Internet, Ethernet, plain old telephone service (POTS) line, public switch telephone network (PSTN), integrated services digital network (ISDN), digital subscriber line (DSL), coaxial cable, fiber optics, satellite, mobile, wireless (e.g. Wi-Fi, WiMAX), SS7 signaling network, fixed line, local area network, wide area network, and others, including any combination of these.

The computing device 300 described above is meant to be an example only. Other suitable embodiments of the controller 122 can also be provided, as it will be apparent to the skilled reader.

Referring now to Fig. 4, there is shown a flow chart of a method 400 for ventilating a patient's airway using the ventilator 100 of Fig. 1. Reference to the ventilator 100 of Fig. 1 will be made in the following paragraphs for ease of reading. It is intended that the controller 122 can have one or more software application(s) configured to perform at least some steps of the method 400.

At step 402, the conduit 114 is connected to the patient's airway 112. This step can be performed by health professional(s) in some embodiments. In some other embodiments, the step 402 can be performed by a suitably programmed robotized machine (not shown).

At step 404, the airway pressure Paw of the patient's airway 112 is monitored. More specifically, the pressure sensor(s) 120 generate(s) airway pressure values Pi over time. The airway pressure values Pi can be communicated to the controller 122 via raw signals, processed signals, raw data, processed data, or any combination thereof. In any case, the controller 122 may receive the airway pressure values Pi measured overtime. The airway pressure values Pi can be stored on a local memory system of the controller 122 for later comparison with one or more airway pressure thresholds Pthres in some embodiments. In some other embodiments, the airway pressure values Pi may be compared to the airway pressure threshold(s) Pthres on the go in some embodiments, after which they may be deleted or stored on a memory system.

At step 406, a first ventilation cycle of air is delivered to the patient's airway 112, which generates pressure variations in the airway 112. In step 406, the first ventilation cycle of air is defined by a first tidal volume Vt1. The first tidal volume Vt1 is delivered by moving the gas delivery element 116 from the start position Ps to the end position Pe, thereby delivering a difference between the corresponding start and end volumes Vs ad Ve of the gas delivery element 116.

Should the monitored airway pressure Paw does not exceed a predetermined airway pressure threshold Pthres, step 406 may be repeated any given number of times to suitably ventilate the patient's airway 112.

At step 408, contingent upon the airway pressure Paw exceeding an airway pressure threshold Pthres during step 406, a second ventilation cycle of air is delivered to the patient's airway 112 subsequent to the delivery of the first ventilation cycle. In step 408, the second ventilation cycle of air is defined by a second tidal volume Vt2 which is lesser than the first tidal volume Vt1, i.e., Vt2 < Vt1. By delivering a lesser volume of air to the patient's airway 112 during one or more consecutive ventilation cycles of air, it is expected that the monitored airway pressure Paw may eventually stop rising, and/or ultimately decrease back under the airway pressure threshold Pthres.

In some embodiments, the gas delivery element 116 may be interrupted during the first ventilation cycle of air in response to the airway pressure Paw exceeding the airway pressure threshold Pthres. For instance, as soon as the controller 122 monitors that the airway pressure threshold Pthres is produced, air delivery may be immediately interrupted to avoid damaging the patient's airway 112. In such circumstances, air evacuation of that ventilation cycle may be performed, after which the patient's airway 112 may be ventilated using either a regular or a modified ventilation cycle of air. It is noted that this step is only optional, as a given ventilation cycle of air may be completed even though the airway pressure threshold Pthres is produced during the given ventilation cycle of air.

In some embodiments, the second tidal volume Vt2 may be maintained for a given number of subsequent ventilation cycles once the airway pressure threshold Pthres has been produced. For instance, in an embodiment where the airway pressure threshold Pthres is met during a first ventilation cycle of air, the subsequent, second ventilation cycle of air may be set to deliver the reduced, second tidal volume Vt2. The reduced, second tidal volume Vt2 may be delivered during a third ventilation cycle of air subsequent to the second ventilation of air; during a fourth ventilation cycle of air subsequent to the third ventilation of air, and so forth.

In some embodiments, the second tidal volume Vt2 may be further reduced between the second ventilation cycle and a successive third ventilation cycle upon determining that the airway pressure threshold Pthres is met again during the second ventilation of air. For instance, if the airway pressure threshold Pthres is met again during the second ventilation cycle of air, the second tidal volume Vt2 may be reduced to a third tidal volume Vt3 being lesser than the second tidal volume Vt2, i.e., Vt3 < Vt2. As such, the third tidal volume Vt3 may be delivered to the patient's airway 112 during the third ventilation cycle of air. It is noted that the tidal volume Vt to be delivered should not be reduced below a given minimal tidal volume Vtmin which is generally associated to a corresponding patient.

In some embodiments, as soon as the monitored airway pressure Paw goes below the airway pressure threshold Pthres during a given ventilation cycle, the tidal volume Vt to be delivered during a subsequent ventilation cycle may be increased.

In some embodiments, the controller 122 may be configured for incrementally reduce a tidal volume Vt of a preceding ventilation cycle upon determining that the monitored airway pressure Paw meet the airway pressure threshold Pthres. The tidal volume Vt1 may be reduced by increment ΔV1, from a first tidal volume Vt1 to a second tidal volume Vt2 where Vt2 = Vt1 - ΔVt1, from a second tidal volume Vt2 to a third tidal volume Vt3 where Vt3 = Vt2 - ΔVt1, and so forth. It is expected that the tidal volume Vt to be delivered may be reduced until a minimal tidal volume Vtmin is reached. For instance, in embodiments where Vt3 < Vtmin, the tidal volume Vt to be delivered may never reach the third tidal volume Vt3 as it would be below the minimal tidal volume Vtmin.

When the monitored airway pressure Paw goes back below the airway pressure threshold Pthres, the controller 122 may be configured for incrementally increase a tidal volume Vt of a preceding ventilation cycle, until the initial, first tidal volume Vt1 is reached. The tidal volume Vt may be increased by increment ΔVt2, from a fifth tidal volume Vt5 to a fourth tidal volume Vt4 where Vt5 = Vt4 + ΔVt2, from the fourth tidal volume Vt4 to a third tidal volume Vt3 where Vt4 = Vt3 + ΔVt2, and so forth, until the initial, first tidal volume Vt1 is reached. In some embodiments, increments ΔVt1 and ΔVt2 are similar to one another. In some other embodiments, increments ΔVt1 and ΔVt2 may be different from one another.

It is noted that the difference between the first and second tidal volumes Vt1 and Vt2 may depend on a difference between the monitored airway pressure Paw, e.g., its maximal value Paw,max throughout a current ventilation cycle, and the airway pressure threshold Pthres. For instance, the more the monitored airway pressure Paw exceeds the airway pressure threshold Pthres, the more the tidal volume Vt to be delivered in a subsequent ventilation cycle may be reduced.

It is noted that the controller may be configured to generate alarm(s) whenever the airway pressure threshold Pthres is produced. Moreover, the alarm(s) may remain activate as long as the tidal volume Vt to be delivered is below the initial, first tidal volume Vt1. The alarm(s) may be displayed on a user interface, be transmitted over a network, and/or be stored for later consultation, depending on the embodiment.

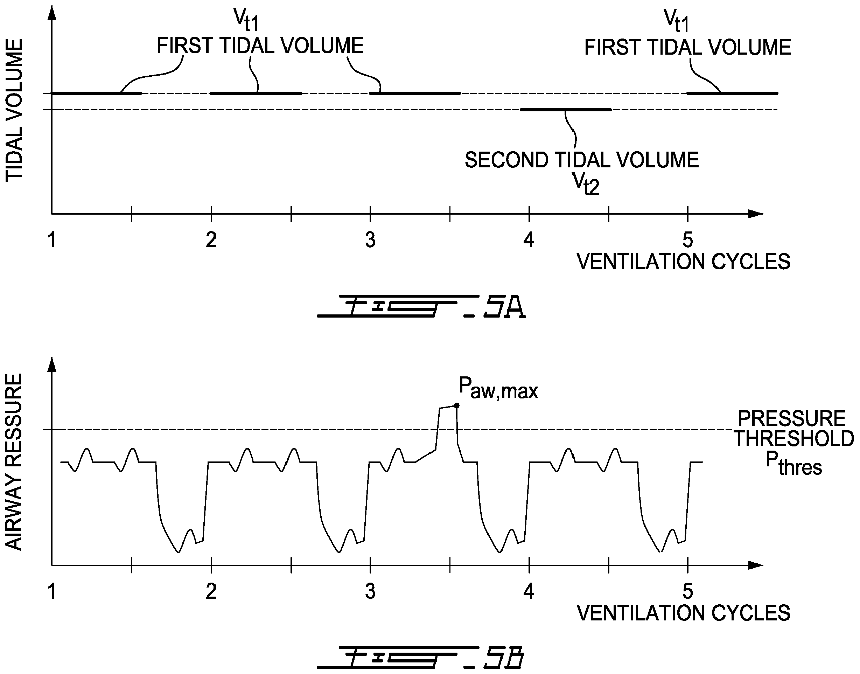

Steps 404, 406 and 408 and some of the optional steps described above are schematically shown in Figs. 5A and 5B which plot the delivered tidal volume Vt and monitored airway pressure Paw, respectively, during a sequence of a few ventilation cycles. As illustrated, a first tidal volume Vt1 is delivered by the ventilator during a first ventilation cycle. During the first ventilation cycle, the monitored airway pressure Paw remains below the airway pressure threshold Pthres. A similar behaviour is noted for a second ventilation cycle subsequent to the first ventilation cycle. For at least some reasons, when the first tidal volume Vt1 is delivered to the patient's airway during a third ventilation cycle of air subsequent to the second ventilation cycle of air, the monitored airway pressure Paw momentarily exceeds the airway pressure threshold Pthres. At this point, the sudden increase in airway pressure Paw is detected by the controller which reduces the first tidal volume Vt1 to a second tidal volume Vt2 being lesser than the first tidal volume Vt1. As such, the reduced, second tidal volume Vt2 is thus set to be delivered to the patient's airway in the following fourth ventilation cycle of air. As discussed above, increasing the delivered tidal volume back to the first tidal volume Vt1 may be decided upon detecting that the monitored airway pressure Paw remains under the airway pressure threshold Pthres during the entirety of the fourth ventilation cycle of air. Still referring to Figs. 5A and 5B, in view of the monitored airway pressure Paw remaining below the airway pressure threshold Pthres during the fourth ventilation of air, the controller increases the second tidal volume Vt2 back to the first tidal volume Vt1 for a fifth ventilation cycle of air subsequent to the fourth ventilation of air.

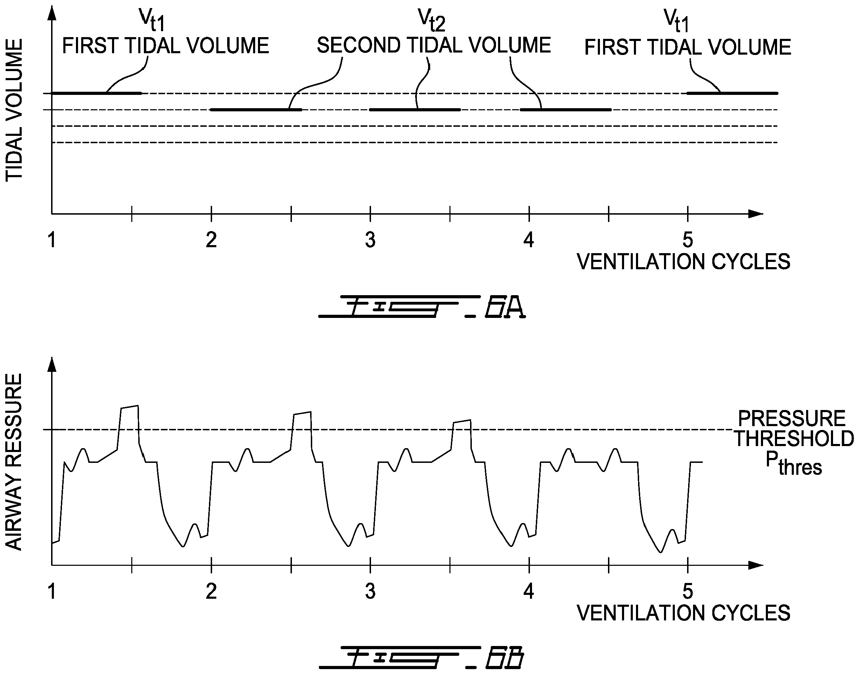

In some embodiments, the reduced, second tidal volume Vt2 can be maintained between said second ventilation cycle and one or more successive ventilation cycles when the airway pressure Paw still exceeds the pressure threshold Pthres in the second ventilation cycle. Referring now to Figs. 6A and 6B, there is shown an example in which the airway pressure Paw exceeds the airway pressure threshold Pthres in a first ventilation cycle. As shown, the first tidal volume Vt1 is reduced to the second tidal volume Vt2 which is delivered to the conduit for the second ventilation cycle, subsequent to the first ventilation cycle. However, in this example, the monitored airway pressure Paw during the second ventilation cycle is still above the airway pressure threshold Pthres. Accordingly, the reduced, second tidal volume Vt2 is maintained for the third ventilation cycle, subsequent to the second ventilation cycle. Still, the monitored airway pressure Paw during the third ventilation cycle may still be above the airway pressure threshold Pthres. Accordingly, the reduced, second tidal volume Vt2 may be maintained for the fourth ventilation cycle, subsequent to the third ventilation cycle. As shown, during the fourth ventilation cycle, the airway pressure Paw decreases below the airway pressure threshold Pthres. Upon this finding, the controller can increase the second tidal volume Vt2 back to the first tidal volume Vt1 and continue the delivery of the first tidal volume Vt1 in subsequent ventilation cycles until the airway pressure threshold Pthres is produced again.

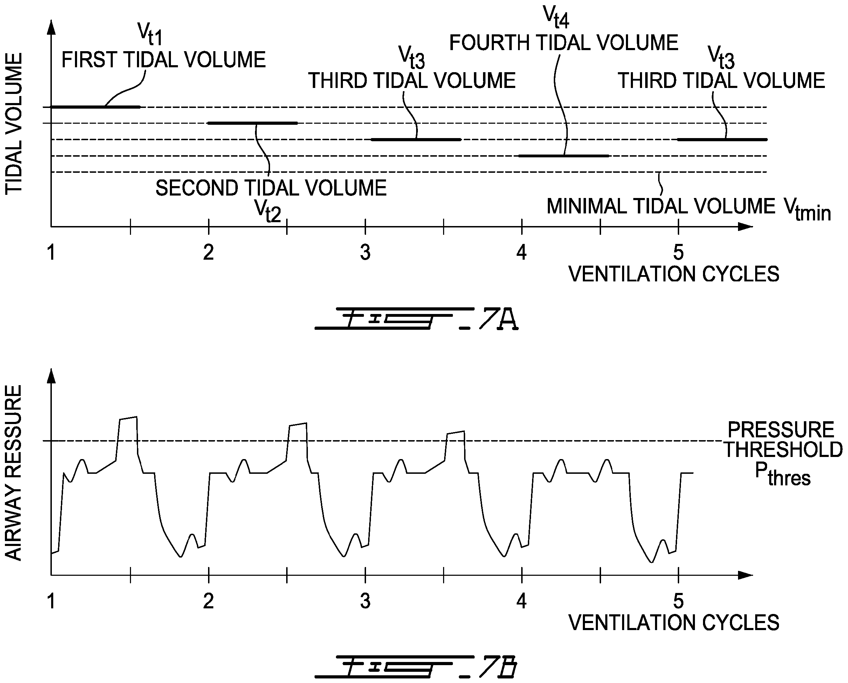

In the embodiment illustrated in Figs. 7A and 7B, the tidal volume Vt to be delivered to the patient's airway is incrementally reduced and increased based on the monitored airway pressure Paw. As depicted, a first tidal volume Vt1 is delivered to the conduit during a first ventilation cycle. In the first ventilation cycle, the monitored airway pressure Paw is monitored to be above the airway pressure threshold Pthres. Accordingly, in the subsequent, second ventilation cycle, a second tidal volume Vt2 being lesser than the first tidal volume Vt1 is delivered to the conduit. The monitored airway pressure Paw is monitored to be above the airway pressure threshold Pthres during the second ventilation cycle. Still, in the subsequent, third ventilation cycle, the tidal volume Vt to be delivered to the conduit is further reduced to a third tidal volume Vt3 being lesser than the first and second tidal volumes Vt1 and Vt2 delivered in the first and second ventilation cycles, respectively. In this specific embodiment, the monitored airway pressure Paw is monitored to be above the airway pressure threshold Pthres during the third ventilation cycle as well. As such, in the subsequent, fourth ventilation cycle, the tidal volume Vt to be delivered to the conduit is further reduced to a fourth tidal volume Vt4 being lesser than the first, second and third tidal volumes Vt1, Vt2 and Vt3 delivered in respective ones of the first, second, and third ventilation cycles. As the monitored airway pressure Paw goes below the airway pressure threshold Pthres during the fourth ventilation cycle, the controller may increase the tidal volume Vt to be delivered in a subsequent ventilation. For instance, the tidal volume Vt to be delivered in the fifth ventilation cycle of air may be any one of the first, second and third tidal volumes Vt1, Vt2 and Vt3. In this specific example, the tidal volume Vt to be delivered is increased in an incremental manner until the initial, first tidal volume Vt1 is reached. More specifically, the third tidal volume Vt3 will be delivered in a fifth ventilation cycle, the second tidal volume Vt2 will be delivered in a sixth ventilation cycle (not shown), and finally, if the monitored airway pressure Paw does not meet the airway pressure threshold Pthres, the first tidal volume Vt1 will be delivered in a seventh ventilation cycle (not shown). In this example, the increment with which the tidal volume Vt is reduced is the same as the increment with which the tidal volume Vt is increased.

In some embodiments, the tidal volume Vt can be reduced in an incremental manner until a given minimal tidal volume Vtmin is reached. For example, in the example shown in Figs. 7A and 7B, the fourth tidal volume Vt4 could not have been reduced as it would have reached the a minimal tidal volume Vtmin below which the patient's airway would be insufficiently ventilated.

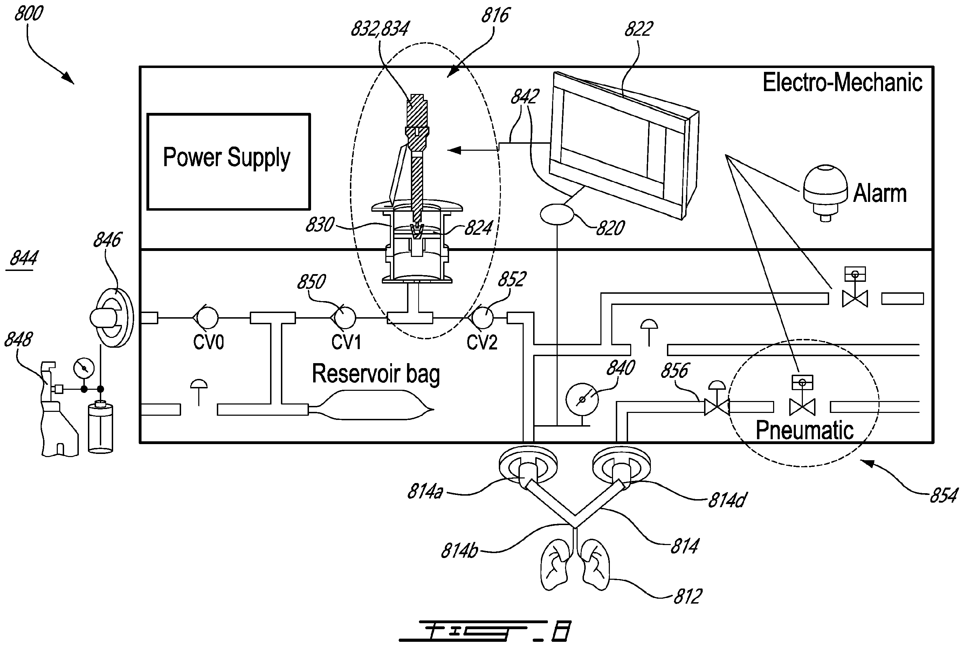

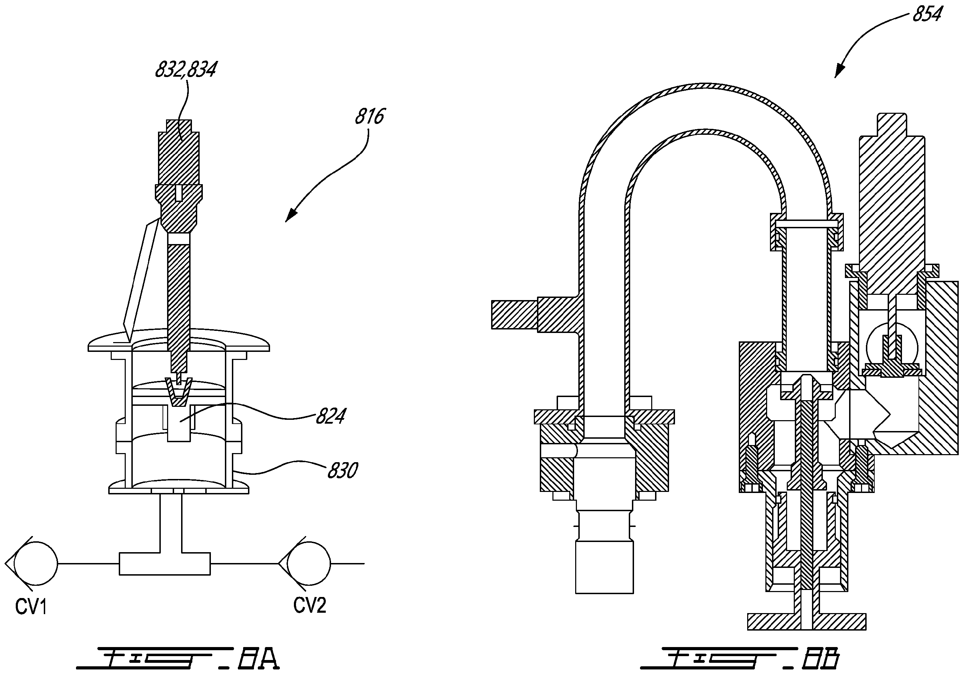

Fig. 8 shows another example of a ventilator 800 to ventilate an airway 812 of a patient, in accordance with one or more embodiments. As depicted, the ventilator 800 has a conduit 814 which is in this case provided in the form of a Y-piece having a fresh air inlet 814a and a fresh air outlet 814b. As shown, the fresh air outlet 814b is connected to a patient's airway 812.

The ventilator 800 has a gas delivery element 816 which is in fluid communication with the fresh air inlet 814a of the conduit 814. As shown, the gas delivery element 816 is configured to deliver air in a sequence of ventilation cycles as described above. The gas delivery element 816 has a cylinder 830 within which a piston 824 moves. By moving the piston 824 from a start position to an end position, a corresponding tidal volume Vt can be delivered to the patient's airway 812. In this specific example, the gas delivery element 816 has an actuator 832, e.g., an electrical linear actuator 834, which is mechanically coupled to the piston 824, and sealed relative to the cylinder 830. The actuator 832 can move the piston 824 in a sequence of back and forth at different axial positions based on an electrical signal. Fig. 8A shows an enlarged view of the gas delivery element 816. It is encompassed that modifying a volume to be delivered to the patient's airway may be advantageous compared to existing ventilators which use a breathable gas source. Indeed, with existing ventilators, excess pressure may be reduced using a release valve. However, opening such a release valve may lead to breathable gas loss, which may be costly overtime. By using such a gas delivery element, no breathable gas is lost via the opening of a release valve. Instead, a reduced amount of fresh air is drawn from the surrounding environment in a subsequent ventilation cycle.

Referring back to Fig. 8, the ventilator 800 has a pressure sensor 820 configured to monitor pressure Paw within the conduit 814. In this example, the pressure sensor 820 is a single pressure sensor which is located upstream from the fresh air inlet 814a. The pressure sensor 820 has a gauge 840 displaying the instantaneous pressure value within the conduit 814. The pressure sensor 820 generates signal(s) and/or data indicative of the instantaneous pressure value.

A controller 822, in this example provided in the form of a computer, is also provided. As shown, the controller 822 and the pressure sensor 820 are communicatively coupled to one another via wired connections 842. The signal(s) and/or data generated by the pressure sensor 820 in real time are communicated to the controller 822 which may locally or remotely process, compare and/or store them as they are received. The controller 822 is also communicatively coupled to the gas delivery unit 816 via a wired connection 842 to control the actuator, for instance.

As discussed above, depending on whether the instantaneous pressure value exceeds a given airway pressure threshold, which may be stored on a memory of the controller, the gas delivery unit 816 is configured to reduce a tidal volume Vt to be delivered to the patient's airway 812, in order to avoid unnecessary and potentially damaging pressure buildup within the patient's airway 812.

The ventilator 800 draws fresh air from the surrounding environment 844, which may be filtered using a fresh air filter 846. As shown, an oxygen source 848 may be used to increase the oxygen content of the surrounding fresh air that is drawn by the ventilator 800. As such, when the piston 824 from the gas delivery element 816 is pulled backward, fresh air from the surrounding environment 844, and also oxygenated by the oxygen source 848, may be drawn within the ventilator 800, and more specifically within a given portion of the cylinder 830, ready to be delivered into the patient's airway 812 by pushing the piston 824 in an opposite direction towards the conduit 814. A first check valve 850 upstream from the gas delivery unit 816 may be used to control entry of fresh air within the ventilator 800. A second check valve 852 downstream from the gas delivery unit 826 may be used to prevent air from reaching the gas delivery unit 816 once it has been breathed or otherwise used by the patient's airway 812. A solenoid valve 854 may be provided downstream from the conduit 814, and more specifically downstream from the used air outlet 814d of the Y-piece conduit. The solenoid valve 854 may be operated to be either closed, thereby favoring a fresh air flow between the gas delivery unit 816 and the patient's airway 812, or open, thereby favoring a used air flow between the patient's airway 812 and a remainder of the ventilator 800 or ultimately the surrounding environment 844, where used air is to be evacuated. An enlarged view of the solenoid valve 854 is shown in Fig. 8B.

Referring back to Fig. 8, a PEEP valve 856 may also be provided downstream from the used air outlet 814d and upstream from the solenoid valve 854. The PEEP valve 856 may be a spring loaded valve that the patient's airway 812 may exhale against. The PEEP valve 856 may prevent ventilator induced lung injury. Additional air filter may be provided upstream from the fresh air inlet and downstream from the used air outlet, as shown in this specific embodiment.

The embodiments described in this document provide non-limiting examples of possible implementations of the present technology. Upon review of the present disclosure, a person of ordinary skill in the art will recognize that changes may be made to the embodiments described herein without departing from the scope of the present technology. Further modifications could be implemented by a person of ordinary skill in the art in view of the present disclosure, which modifications would be within the scope of the present technology.

## Claims

1. A ventilator (100; 800) to ventilate an airway (112; 812) of a patient, the ventilator (100; 800) comprising:
a conduit (114; 814) having an inlet (114a; 814a) and an outlet (114b; 814b), said outlet (114b; 814b) being configured to be connected to said airway (112; 812);
a gas delivery element (116; 816) in fluid communication with said inlet (114a; 814a) of said conduit (114; 814), said gas delivery element (116; 816) configured to deliver air in a sequence of ventilation cycles, each ventilation cycle being defined by a corresponding tidal volume to be delivered to said airway (112; 812) via said conduit (114; 814), each tidal volume corresponding to a difference between a start volume and an end volume of said gas delivery element (116; 816) for that ventilation cycle;
a pressure sensor (120; 820) configured to monitor pressure within said conduit (114; 814);
a controller (122; 822) communicatively coupled to said gas delivery element (116; 816) and said pressure sensor (120; 820), said controller (122; 822) having a processor (302) and a non-transitory memory (304) having stored thereon instructions (308) that when executed by said processor (302) perform the step of reducing said tidal volume between a first ventilation cycle and a successive second ventilation cycle contingent upon said pressure exceeding a pressure threshold in said first ventilation cycle.

2. The ventilator (100; 800) of claim 1, wherein said gas delivery element (116; 816) has a moving element (124) displaceable between a start position corresponding to said start volume and an end position corresponding to said end volume.

3. The ventilator of claim 2, wherein said moving element is a piston (128, 824) moving within a cylinder (130; 830), said start and end positions corresponding to axial positions of said cylinder (130; 830).

4. The ventilator (100; 800) of claim 1, 2 or 3, wherein said controller (122; 822) is configured for interrupting said gas delivery element (116; 816) during said first ventilation cycle in response to said pressure exceeding said pressure threshold.

5. The ventilator (100; 800) of any preceding claim, wherein said controller (122; 822) is configured for maintaining said reduced tidal volume between said second ventilation cycle and a successive third ventilation cycle contingent upon said pressure exceeding said pressure threshold in said second ventilation cycle.

6. The ventilator (100; 800) of any of claims 1 to 4, wherein said controller (122; 822) is configured for further reducing said tidal volume between said second ventilation cycle and a successive third ventilation cycle contingent upon said pressure exceeding said pressure threshold in said second ventilation cycle.

7. The ventilator (100; 800) of any of claims 1 to 4 or 6, wherein said controller (122; 822) is configured for incrementally reducing a tidal volume of a preceding ventilation cycle contingent upon said pressure exceeding said pressure threshold in that preceding ventilation cycle, said reducing is performed until a minimal tidal volume is reached.

8. The ventilator (100; 800) of any preceding claim, wherein said controller (122; 822) is configured for increasing said reduced tidal volume between said second ventilation cycle and a successive third ventilation cycle contingent upon said pressure being below said pressure threshold in said second ventilation cycle.

9. The ventilator of any preceding claim, wherein said controller is configured for incrementally increasing a tidal volume of a preceding ventilation cycle contingent upon said pressure being below said pressure threshold in that preceding ventilation cycle, said increasing being performed until said tidal volume of said first ventilation cycle is reached.

10. The ventilator (100; 800) of any preceding claim, further comprising a user interface (306) communicatively coupled to said controller (122; 822), said user interface (306) receiving an input indicative of said first tidal volume.

11. A computer program product stored in a non-transitory memory (304) of a controller (122) further having a processor (302), the computer program product having computer readable instructions which, when executed by the processor (302) controls a patient ventilator (100) based on airway pressure monitoring when executed by the controller (122), including the step of: reducing a tidal volume between a first ventilation cycle and a successive second ventilation cycle contingent upon an airway pressure measurement exceeding a pressure threshold during said first ventilation cycle.
